Europäisches Patentamt

European Patent Office

Office européen des brevets

(19)

(11) Publication number: **0 074 748**
**B1**

(12) EUROPEAN PATENT SPECIFICATION

(45) Date of publication of patent specification: **20.05.87**

(21) Application number: **82304552.1**

(22) Date of filing: **27.08.82**

(51) Int. Cl.⁴: **A 61 H 33/06,** F 24 C 7/04, F 24 D 13/02

(54) Sauna.

(30) Priority: **01.09.81 JP 129969/81**
**07.12.81 JP 182648/81**

(43) Date of publication of application:
**23.03.83 Bulletin 83/12**

(45) Publication of the grant of the patent:
**20.05.87 Bulletin 87/21**

(84) Designated Contracting States:
**DE FR GB**

(56) References cited:
**DE-B-1 032 439**
**FR-A-2 246 111**
**US-A-3 564 200**
**US-A-3 875 596**

(73) Proprietor: **YOSHIHARA & CO. LTD.**
**17-4, Shibuya-1-chome Shibuya-ku**
**Tokyo (JP)**

(72) Inventor: **Yoshihara, Kenjiro**
**17-4, Shibuya-1-chome**
**Shibuya-ku Tokyo (JP)**

(74) Representative: **Barlow, Roy James et al**
**J.A.KEMP & CO. 14, South Square Gray's Inn**
**London WC1R 5EU (GB)**

Courier Press, Leamington Spa, England.

## Description

### Background of the Invention

This invention relates to saunas, and more particularly it is concerned with a novel sauna utilizing infrared radiation which can be readily installed at low cost for general household use and have effects in improving health and developing bodily gracefulness.

Saunas have been used from olden times because saunas have the effects of improving the circulation of the blood and promoting metabolism. Essentially, a conventional sauna consists in generating extremely hot, relatively dry air by a heating source, such as heated stones, to provide an atmosphere of 80—120°C, and exposing a human body to the atmosphere. However, expensive equipment is necessary for creating such atmosphere of high temperature, and installation of the equipment is not easy, so that difficulties are faced with in installing a sauna for general household use. Also, the process for availing oneself of the benefits offered by this type of sauna is relatively complex. Moreover, since the sauna basically consists in exposure to an atmosphere of high temperature, it not lend itself for use by the aged, children and those who suffer from high blood pressure, heart trouble, etc.

US—A—3,875,596 discloses a small collapsible sauna comprising a cabin enclosing a heat source intended to raise the air temperature in the cabin up to the conventional range of 60°C to 70°C encountered in saunas. The heat source includes an electric heater and an air blower.

### Summary of the Invention

A main object of the invention is to provide a sauna capable of being installed at low cost and with ease for general household use and which can be utilized without any trouble by the aged, children and those who suffer from high blood pressure, heart trouble, etc.

The present invention provides a sauna room including: a bath tub or a shower to provide a source of hot water for the user; a heat source comprising an infrared radiation generator having (a) an electric heater, (b) an infrared radiation-generating surface having a ceramic particle layer adapted to be heated by said electric heater for generating infrared radiation, and (c) means associated with said electric heater for controlling the temperature thereof in such a manner that said infrared generating surface is maintained at a temperature of not greater than about 80°C; and a cabin for the user surrounding the heat source.

Such a generator is for example disclosed in US—A—3,453,413. The generator is preferably adapted to generate far infrared radiation of about 8—12 microns in wavelength. The generator is preferably installed on a side wall of the bathroom.

The cabin may include a top plate and a curtain depending from said top plate, and alternatively may include a foldable framework, and a transparent sheet of synthetic resin enclosing said framework.

The sauna room according to the invention preferably further comprises a protector for said infrared radiation generating surface of said generator.

### Brief Description of the Drawings

Fig. 1 is a schematic top plan view of an embodiment of a sauna according to the invention;

Fig. 2 is a schematic view of the infrared radiation generator and the cabin of the sauna shown in Fig. 1;

Fig. 3 is a sectional view of an infrared radiation generator of the sauna shown in Fig. 1;

Fig. 4 is a front view of an infrared radiation generator combined with a protector;

Fig. 5 is a side view of the infrared radiation generator and the protector shown in Fig. 4; and

Fig. 6 is a schematic perspective view of an alternative form of the cabin.

### Description of the Preferred Embodiments

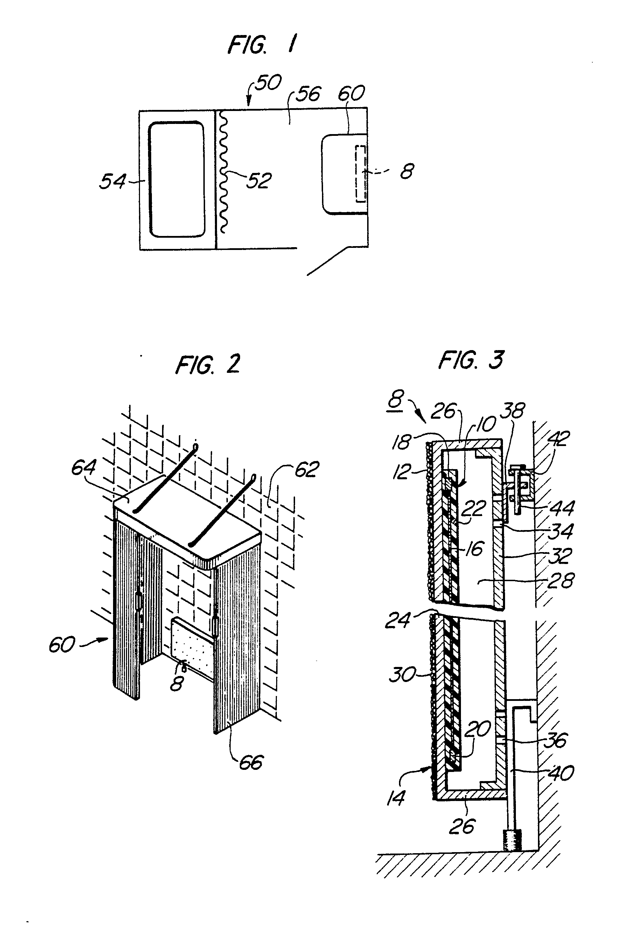

Referring to Figs. 1 and 2, a sauna comprising an embodiment of the invention utilizing a Western style bathroom is generally designated by the reference numeral 50.

The sauna generally designated by the reference numeral 50 comprises a bathroom 56 having a bath tub 54 partitioned off the rest of the bathroom by a curtain 52, an infrared radiation generator 8 installed in the bathroom 56, and a tent or cabin 60 mounted to surround the infrared radiation generator 8 while being freely opened and closed. The detailed construction of the infrared radiation generator 8 will be described with reference to Fig. 3.

Referring to Fig. 2, the cabin 60 comprises a top panel 64 hung from a side wall 62 of the bathroom 56, and a curtain 66 depending from the top panel 64 and having an edge portion movable through a runner metal member, not shown, so as to open and close the curtain 66.

In this way a compact sauna room can be readily obtained by bringing the curtain 66 of the cabin 60 to a closed position.

The atmospheric temperature is preferably raised to the range of about 32—35°C, namely higher than the temperature at which the human body begins to perspire, prior to exposure to the infrared radiation by the infrared radiation generator 8, to obtain satisfactory perspiration by irradiation of the infrared radiation. However, in the case of a western style bath in which the bathtub is partitioned off from the rest of the bathroom by the curtain, it would be difficult to raise the atmospheric temperature to the aforesaid level. This problem can be eliminated by closing the curtain 66 to provide a compact sauna room, because the atmospheric temperature in the sauna room can be raised to the desired range of about 32—35°C merely by effecting heating by means of the infrared radiation generator 8.

The construction having the cabin 60 can achieve excellent results for similar reasons when applied to a bathroom having a relatively large volume as compared with its bathtub.

Referring to Fig. 3 the infrared radiation generator 8 comprises an electric heater 10, and an infrared radiation generating surface 14 including a ceramic particles layer 12 adapted to be heated by the heater 10 for generating infrared radiation.

The electric heater 10 comprises a sheet-like electric heat generating element 16 in the form of a glass fiber coated with a carbon heat generator, for example. The element 16 has electrodes 18 and 20 connected to opposite edge portions thereof which in turn are connected through wires, not shown, to a power source, not shown, to have a supply of a current therefrom. The sheet-like electric heat generating element 16 has attached to opposite sides thereof protective and insulating layers 22 each having a sheet of a synthetic resin such as polyester, adhered to the entire surface thereof, so as to give required strength to the heater 10 and electrically insulate the element 16 from external humidity. The electric heater 10 has attached thereto a switch, a thermostat and a fuse, all not shown. The electric heater 10 is controlled by the thermostat in such a manner that the temperature of the infrared radiation generating surface 14 does not exceed about 80°C.

The infrared radiation generating surface 14 includes a front panel 24 preferably of sheet steel having an outer surface coated with the ceramic particles layer 12 and an inner surface to which the electric heater 10 is adhered and secured. The front panel 24 has side panels 26 formed integrally therewith to define a space 28 therein.

The ceramic particles layer 12 includes ceramic particles 30 adhered and secured to the outer surface of the front panel 24 by an adhesive agent which is heat resistant. When heated by the electric heater 10, the ceramic particles 30 emit infrared radiation of about 8 to 12 microns in wavelength. The ceramic particles 30 are preferably coarse siliceus particles such as silica sand. However, other infrared radiation generating ceramic particles such as of the zirconium base, titanium base, alumina base, etc. may be used. The heat resistant adhesive agent may be preferably an epoxy resin base adhesive agent, and bonding of the front panel 24 to the heater 10 may be effected by the same adhesive agent. One example of the ceramic particles layer 12 is disclosed in the said US—A—3,453,413.

The side panels 26 have a rear panel 32 secured thereto by suitable fastening means such as screws to close the space 28. The rear panel 32 is formed with upper and lower ventilating apertures 34 and 36, to allow air heated in the space 28 to flow out through the upper apertures 34 while allowing outside air to flow into the space 28 through the lower apertures 36.

The rear panel 32 has a pair of upper mounting metal members 38 and a pair of lower mounting metal members 40 secured thereto by suitable fastening means such as screws. The upper pair of mounting plate members 38 each comprises a plate member of the L-shape in cross-section, and one of the plate portions thereof is located between upper and lower plate portions of a mounting metal member 42 of a U-shape cross section secured to a side wall of the bathroom 56 by suitable fastening means such as screws, and a pin 44 extends through the upper and lower plate portions of the member 42 and the plate portion of the member 38. The lower pair of mounting metal members 40 each comprises a plate member of the shape of an inverted letter J, and a longer plate portion or leg thereof abuts against the floor of the bathroom 56, and a shorter plate portion thereof abuts against the side wall of the bathroom. Thus, the infrared radiation generator 8 is installed in the bathroom 56 with a predetermined distance from the side wall thereof and a predetermined elevation from the floor thereof.

Operation of the sauna of the aforesaid construction will now be described. The user of the sauna 50 turns on the switch of the infrared radiation generator 8 after or before or simultaneously as he puts water in the bathtub 54 and heats same or places hot water therein to be ready for bathing in the bathtub 54. In the infrared radiation generator 8, the infrared radiating generating surface 14 is heated to a temperature of about 80°C by the electric heater 10, and infrared radiation or rays of about 8—12 microns in wavelength are emitted from the ceramic particles layer 12. The air in the space 28 is heated by the heater 10 and the heated air flows through the ventilating apertures 34 to outside. The atmosphere in the bathroom 56 is heated by the hot water in the bathtub 54, the infrared radiation emanating from the generator 8 and the heated air from the ventilating apertures 34 and the temperature rises to the range between 32 and 35°C which is above the temperature level at which perspiration is induced in human bodies. Then, the user preferably enters the bathtub 54 and warms his body to promote perspiration, and thereafter, takes a suitable posture in front of the far infrared radiation generating surface 14 to be exposed to the infrared radiation.

The wavelength of about 8—12 microns of the infrared rays emanating from the infrared radiation generator 8 is substantially the same as the infrared rays emanating from the human body of normal temperature. Thus, as the body of the user is exposed to the infrared radiation of this wavelength range, resonance absorption takes place so that about 99% the energy is absorbed by the body, penetrating the body to a depth of about 40 mm below the surface of the skin. This makes it possible to heat the body from the inside even if the atmosphere has a temperature in the range of about 32 and 35°C without raising the temperature at the surface of the skin. This greatly promotes perspiration.

The sauna according to the invention can achieve the effect of causing the user to profusely perspire by the aforesaid action. Additionally, the sauna according to the invention can achieve the following effects:

(1) The sauna can be installed at low cost and with ease for general household use since the infrared generator only needs to be mounted in a usual bathroom.

(2) The atmospheric temperature is about 32—35°C which is relatively low and this enables users to enjoy perspiration without feeling heat and having a choking sensation which users usually feel in conventional saunas in which temperature runs as high as about 80—120°C. Moreover, the atmosphere is not too stimulating, so that even the aged, children and those suffering from high blood pressure, heart trouble, etc. can enjoy a sauna without any trouble.

(3) Penetration of the body by the infrared rays of about 8—12 microns to reach the depth of about 40 mm from the surface of the skin stimulates not only the sudoriparous glands but also the sebaceous glands, so that the secretion of the sebaceous glands passes out through the pores together with perspiration to reduce subcutaneous fat thereby to enable bodily gracefulness to be developed in the true sense of the words while achieving slimming.

(4) The fat content of the secretion from the sebaceous glands through the pores contains injurious heavy metals which are not contained in perspiration, and the fact that the heavy metals can also be released from the body is conductive to improved health.

(5) The infrared radiation penetrating the human body and reaching the depth of about 40 mm from the surface of the skin is absorbed by the body and invigorates the molecular activities of the body cells, thereby achieving a sort of massage effect. This improves metabolism and enables wastes deep below the skin to be secreted through the pores of the sebaceous glands, so that the effects of beautifying the skin and developing bodily gracefulness while invigorating the body can be achieved.

(6) Exposure to the atmospheric temperature of about 32—35°C and the infrared rays of about 8—12 microns is tantamount to exposure to natural sunlight. The result of this is that the parasympathetic of the autonomous nervous system is stimulated to set the mind at rest to remove fatigue from the nerve while expanding the capillaries to improve the functioning of the respiratory organs. This is conducive to elimination of stress to which the mind and body are subjected thereby to enable the user of the sauna to be refreshed both in mind and body.

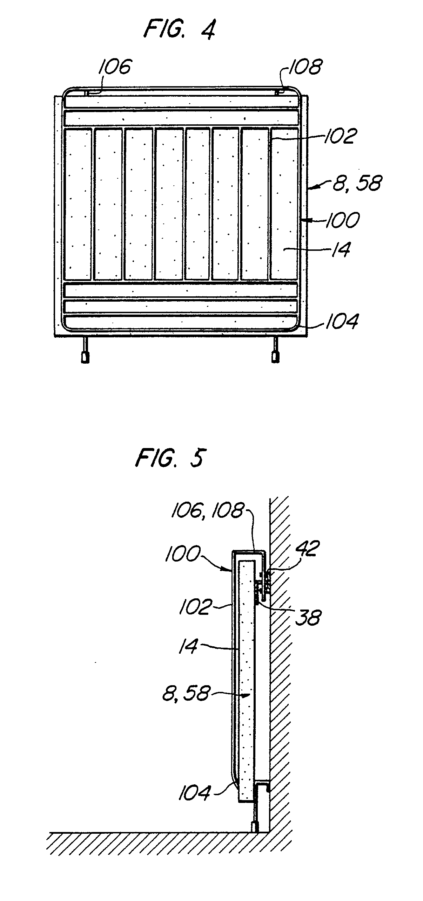

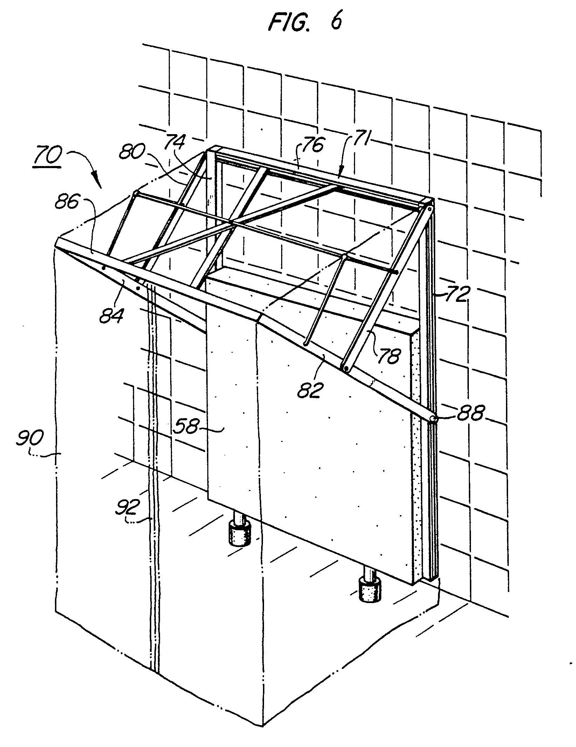

An alternative form of the cabin will be described by referring to Figs. 4 to 6.

Referring to Figs. 4 to 6, the cabin generally designated by the reference numeral 70 comprises a foldable framework 71 including a pair of side frames 72 and 74 secured by suitable fastening means such as screws to opposite sides of the infrared radiation generator 58. The side frames 72 and 74 are connected together at the upper ends by a rear top frame 76. The side frames 72 and 74 have upper ends pivotally connected to support frames 78 and 80 respectively at one end thereof, and the support frames 78 and 80 pivotally support at the other end thereof movable side frames 82 and 84 respectively at their central portions. The movable side frames 82 and 84 are connected together at the upper end thereof by a front top frame 86 and connected at the lower end thereof to the side frames 72 and 74 respectively at 88 for pivotal and sliding movement. The connection at 88 can be achieved by providing each of the side frames 72 and 74 with a guide channel and mounting a guide roller at the lower end of each of the movable side frames 82 and 84, for example, so that the guide roller can move in rolling sliding movement in the guide channel. The frames 76, 78, 80, 82, 84 and 86 are reinforced by auxiliary frames shown in the figures but having no reference numerals. Of the auxiliary frames, those connected to the top frames 76 and 86 can be connected for pivotal and sliding movement, like the connection 88 between the side frames 72 and 74 and the movable side frames 82 and 84. Thus, when the front top frame 86 in its foremost position as is moved rearwardly, the movable side frames 82 and 84 move downwardly at their lower end along the side frames 72 and 74, respectively, so that the movable side frames 82 and 84 reach a position in which their upper ends are close to the upper ends of the side frames 72 and 74 while the front top frame 86 reaches a position in which it is close to the rear top frame 76. Thus, the framework 71 can be folded.

The framework 71 is enclosed by a sheet 90 of synthetic resin in the form of a box without a bottom to provide a sauna room therein. The sheet 90 is preferably a transparent vinyl sheet and has a fastener 92 attached to its front surface to open and close same.

The cabin 70 of this alternative offers the advantages that it is simple to install and it enables a compact size to be obtained when the framework 71 is folded when not in use.

In all the embodiments and alternatives described hereinabove, the infrared radiation generator 8, 58 may be provided with a protector 100 shown in Figs. 4 and 5.

The protector 100 comprises a multiplicity of fine rod members 102 located on the infrared radiation generating surface 14 of the infrared radiation generator 8, 58, and some rod member 102 is bent inwardly toward the surface 14 at its lower end portion to have its lower end positioned against the surface 14. The protector 100 has its upper end secured to connectors 106 and 108 which, as shown in Fig. 5, are inserted in the upper mounting metal members 38 and 42 in place of the pin 44 shown in Fig. 3.

The provision of the protector 100 enables the generating surface 14 of the infrared radiation generator 14 to be protected from damage and ensures that the sauna can be used safely without any accident.

## Claims

1. A sauna room including: a bath tub or a shower (4, 54) to provide a source of hot water for the user; a heat source comprising an infrared radiation generator (8, 58) having (a) an electric heater (16), (b) an infrared radiation-generating surface (30) having a ceramic particle layer (12) adapted to be heated by said electric heater for generating infrared radiation, and (c) means associated with said electric heater for controlling the temperature thereof in such a manner that said infrared generating surface is maintained at a temperature of not greater than about 80°C; and a cabin (60, 70) for the user surrounding the heat source.

2. A sauna room as claimed in Claim 1, characterised in that said infrared radiation generator (8, 58) is adapted to generate infrared radiation of a wavelength of about 8—12 microns.

3. A sauna room as claimed in Claim 1 or Claim 2, characterised in that said infrared radiation generator (8, 58) is installed on a side wall (62) of the sauna room.

4. A sauna room as claimed in any one of claims 1 to 3, characterised in that said cabin (60) includes a top panel (64), and a curtain (66) depending from said top panel.

5. A sauna as claimed in any one of claims 1 to 3, characterised in that said cabin includes a foldable framework (71), and a transparent sheet (90) of synthetic resin enclosing said framework.

6. A sauna as claimed in any one of claims 1 to 5, further characterised by a protector (100) for said infrared radiation generating surface of said generator.

## Patentansprüche

1. Saunaraum mit
— einer Badewanne oder einer Dusche (4, 54), die dem Benutzer eine Quelle heißen Wassers liefert;
— einer Wärmquelle mit einem Infrarotstrahlengerät (8, 58), das
(a) einen elektrischen Heizer (16),
(b) eine Infrarotstrahlen erzeugende Oberfläche (30) mit einer Schicht aus keramischen Partikeln (12) aufweist, wobei die Schicht durch den elektrischen Heizer zum erzeugen von Infrarotstrahlung aufgeheizt wird und
(c) mit dem elektrischen Heizer verbundene Mittel zum Steuern der Temperatur des Heizers derart, daß die Infrarotstrahlen erzeugende Oberfläche auf einer Temperatur gehalten wird, die nicht größer als 80°C ist und mit einer Kabine (60, 70) für den Benutzer, die die Wärmquelle umschließt.

2. Saunaraum nach Anspruch 1, dadurch gekennzeichnet, daı das infrarotstrahlengerät (8, 58) so ausgebildet ist, daß es Infrarotstrahlung einer Wellenlänge von 8 bis 12 μm erzeugt.

3. Saunaraum nach Anspruch 1 oder 2, dadurch gekennzeichnet, daß das Infrarotstrahlengerät (8, 58) an einer Seitenwand (62) des Saunaraums angebracht ist.

4. Saunaraum nach einem Ansprüche 1 bis 3, dadurch gekennzeichnet, daß die Kabine (60) ein Deckenbrett (64) und einen Vorhang (66), der an dem Deckenbrett befestigt ist, aufweist.

5. Sauna nach einem der Ansprüche 1 bis 3, dadurch gekennzeichnet, daß die Kabine einen faltbaren Rahmen (71) und eine transparente Platte (90) aus synthetischem Harz, das den Rahmen umschließt, aufweist.

6. Sauna nach einem der Ansprüche 1 bis 5, gekennzeichnet durch eine Schutzvorrichtung (100) für die infrarotstrahlende Oberfläche des Infrarotstrahlengerätes.

## Revendications

1. Sauna comprenant: une baignoire ou une douche (4, 54) destinée à fournir à l'utilisateur une source d'eau chaude; une source de chaleur comprenant un générateur de rayonnement infrarouge (8, 58) ayant (a) un élément de chauffage électrique (16), (b) une surface génératrice de rayonnement infrarouge (30) comprenant une couche de particules céramiques (12) apte à être chauffée par l'élément de chauffage électrique pour générer un rayonnement infrarouge, et (c) un moyen associé à l'élément de chauffage électrique pour commander la température de ce dernier de façon que la surface génératrice de rayonnement infrarouge soit maintenue à une température ne dépassant pas environ 80°C; et une cabine (60, 70) destinée à l'utilisateur, entourant la source de chaleur.

2. Sauna selon la revendication 1, caractérisé en ce que ledit générateur de rayonnement infrarouge (8, 58) est agencé de façon à produire un rayonnement infrarouge ayant une longueur d'onde d'environ 8 à 12 microns.

3. Sauna selon la revendication 1 ou la revendication 2, caractérisé en ce que ledit générateur de rayonnement infrarouge (8, 58) est monté sur une paroi latérale (62) du sauna.

4. Sauna selon l'une quelconque des revendications 1 à 3, caractérisé en ce que ladite cabine (60) comprend un panneau de toit (64), et un rideau (66) pendant dudit panneau de toit.

5. Sauna selon l'une quelconque des revendications 1 à 3, caractérisé en ce que ladite cabine comprend un châssis pliant (7), et une feuille transparente (90) en résine synthétique renfermant ledit châssis.

6. Sauna selon l'une quelconque des revendications 1 à 5, caractérisé, en ce qu'il comprend en outre un élément de protection (100) pour ladite surface génératrice de rayonnement infrarouge dudit générateur.

0 074 748

FIG. 1

FIG. 2

FIG. 3

1

FIG. 4

FIG. 5

FIG. 6